# FASCICULE DE BREVET EUROPEEN

(11) **EP 0 807 822 B1**
(45) Date de publication et mention de la délivrance du brevet: **05.09.2001**
(21) Numéro de dépôt: 97400899.7
(22) Date de dépôt: 22.04.1997
(51) Int. Cl.: G01N 33/00, G01N 33/24

(54) **Mesure de flux surfacique de gaz**
Messung eines aus einer Oberfläche austretenden Gasstromes
Measurement of the gas flux from a surface

(30) Priorité: 14.05.1996 FR 9605996
(43) Date de publication de la demande: 19.11.1997
(73) Titulaire: INSTITUT NATIONAL DE L'ENVIRONNEMENT INDUSTRIEL ET DES RISQUES, 60550 Verneuil en Halatte (FR)
(72) Inventeur: Tauziede, Christian, 60550 Verneuil-en-Halatte (FR); Pokryszka, Zbigniew, 60550 Verneuil-en-Halatte (FR); Jodart, Alain, Bazicourt, 60700 Pont Ste Maxence (FR)
(74) Mandataire: Rinuy, Santarelli

(56) Documents cités:
- DE-A- 3 409 453
- GB-A- 1 301 021
- US-A- 5 355 739

## Description

La présente invention concerne d'une manière générale la mesure de flux de gaz émis par une surface et s'applique plus particulièrement à la mesure de flux de gaz, par exemple polluants, émis par un terrain, tel que décharge d'ordures ménagères.

Un dispositif connu comporte une chambre en forme de cloche recouvrant une surface et délimitant un volume au-dessus de cette surface. La chambre est alimentée en un gaz vecteur fourni par une bouteille de gaz comprimé. Un conduit relie la chambre à un analyseur. Ce dispositif est utilisé pour mesurer la concentration de gaz émis par la surface.

Le gaz vecteur a un débit très grand par rapport au gaz émis par la surface. Le gaz vecteur et le gaz émis par la surface se mélangent et le gaz émis par la surface est entraîné par le gaz vecteur vers l'analyseur.

Ce dispositif connu est complexe à mettre en oeuvre, en raison de la présence de la bouteille d'air comprimé qui est encombrante, lourde à manipuler et qu'il faut remplacer lorsqu'elle est vide.

De plus, le gaz dont on veut mesurer la concentration est dilué dans la chambre, en raison de l'alimentation en gaz vecteur. Cette dilution provoque une diminution de la concentration de gaz à mesurer, et par conséquent diminue la sensibilité de la mesure. Il est donc nécessaire d'utiliser un analyseur de grande précision pour compenser la diminution de la concentration de gaz à mesurer, si l'on souhaite obtenir une bonne précision.

Cependant, le fonctionnement de ce dispositif provoque une surpression à l'intérieur de la chambre, due à l'alimentation en gaz vecteur. Cette surpression altère la mesure, et ce d'autant plus que la concentration à mesurer est généralement faible, et donc sensible à une perturbation. L'exactitude de la mesure n'est pas parfaite.

Donc, même avec un analyseur de grande précision, la mesure demeure perturbée par la surpression créée par le dispositif, et la précision finalement obtenue avec ce dispositif demeure limitée.

Par ailleurs, ce dispositif doit fonctionner en régime établi, c'est-à-dire après un laps de temps suivant la mise en place du dispositif sur le lieu de mesure, ce qui implique un temps de mesure relativement long, par exemple de quelques dizaines de minutes.

Le document US-A-5,355,739 décrit un dispositif de mesure de flux de gaz émis par la terre comportant une chambre qui couvre la surface de la terre à mesurer et délimite un volume d'une mixture de gaz et d'air. Un échantillon du gaz dans la chambre est envoyé à l'analyseur à travers un conduit.

Le document DE-A-34 09 453 décrit un procédé de mesure de l'humidité contenue dans un matériau solide poreux comportant une chambre qui couvre la surface du matériau et délimite un volume d'air.

La chambre est connectée avec un dispositif analyseur à travers un circuit en boucle fermée pour lequel l'air contenu dans la chambre est recirculé continuellement. La présente invention vise à remédier aux inconvénients de la technique antérieure, en fournissant un dispositif pour la mesure de flux de gaz émis par une surface qui est simple et plus rapide à mettre en oeuvre que les dispositifs connus tout en offrant une précision de mesure accrue.

A cette fin, l'invention propose un dispositif de mesure de flux de gaz émis par une surface, comportant une chambre recouvrant ladite surface, délimitant un volume fermé au-dessus de la surface et reliée à un analyseur de gaz, caractérisé en ce qu'il comporte un moyen de circulation des gaz contenus dans ladite chambre, formant un circuit en boucle fermée entre la chambre et l'analyseur.

Le dispositif remédie aux inconvénients de la technique antérieure, car d'une part sa manipulation et sa mise en place sont facilitées en raison de l'absence de toute bouteille de gaz comprimé et d'autre part son utilisation ne perturbe que faiblement l'émission de gaz que l'on étudie, et fournit donc une mesure plus précise, à précision d'analyseur identique, que les dispositifs connus.

En outre, la mesure est réalisée en régime transitoire, c'est-à-dire dès l'installation du dispositif sur le lieu de mesure, ce qui permet d'obtenir le résultat plus rapidement que selon la technique antérieure.

Le dispositif permet une accumulation de gaz dans la chambre, et une circulation de gaz à l'extérieur de la chambre.

Selon une caractéristique préférée, le moyen de circulation comporte un conduit de circulation entre une entrée formée par une portion perforée s'étendant dans la chambre et une sortie débouchant dans la chambre, via une pompe de circulation de gaz et l'analyseur.

Selon d'autres caractéristiques préférées, la portion perforée s'étend sensiblement entre le centre de la chambre et une paroi latérale de la chambre, et la sortie est située dans une paroi latérale sensiblement en vis à vis de l'entrée. Cet agencement favorise une homogénéisation des gaz à l'intérieur de la chambre. Les gaz sont captés dans une zone de la chambre relativement grande, puis circulent dans l'analyseur et sont ensuite rejetés dans la chambre, en dehors de la zone dans laquelle sont captés les gaz.

Avantageusement, le dispositif selon l'invention comporte un moyen de brassage des gaz contenus dans ladite chambre. Le moyen de brassage comporte un ventilateur actionné par un moteur et favorise également l'homogénéisation des gaz à l'intérieur de la chambre.

Selon une caractéristique de l'invention, la portion perforée est située entre le moyen de brassage et la surface. Ainsi, le brassage ne perturbe pas le passage des gaz émis par la surface vers la portion perforée.

Avantageusement, la chambre comporte un trou de communication avec l'extérieur. Le trou est situé dans une paroi de la chambre sensiblement opposée à la surface. Ce trou limite la surpression à l'intérieur de la chambre.

Corrélativement, l'invention propose un procédé de mesure de flux de gaz émis par une surface comportant l'application d'un facteur correctif aux mesures réalisées, ce qui permet de tenir compte de l'influence du dispositif de mesure sur les valeurs mesurées.

Les caractéristiques et avantages de la présente invention apparaîtront plus clairement à la lecture d'un mode de réalisation illustré par les dessins ci-joints, dans lesquels :
- la figure 1 est une vue schématique du dispositif selon l'invention, et
- la figure 2 est un exemple de résultats obtenus grâce à l'invention.

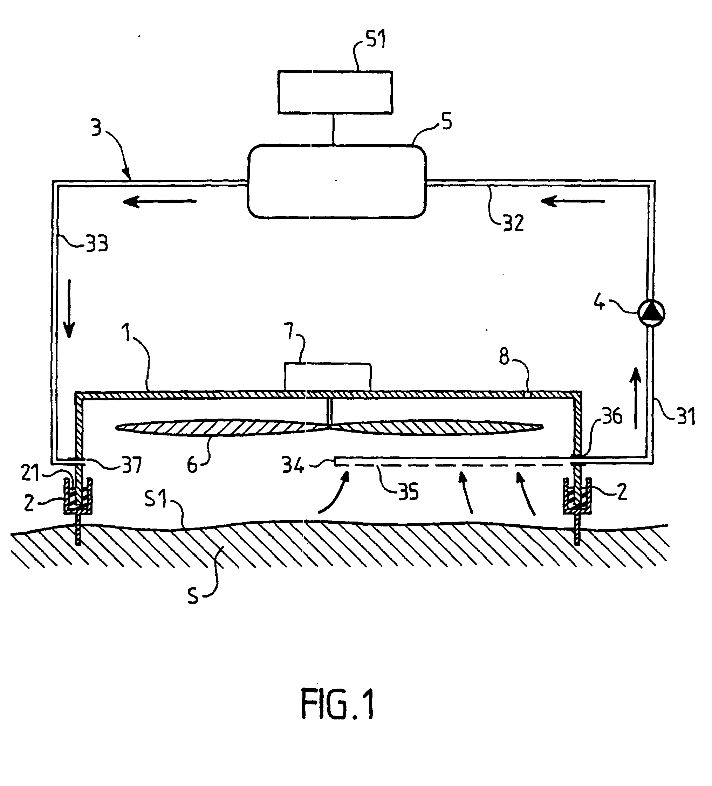

En référence à la **figure 1,** le dispositif de mesure de flux de gaz selon l'invention comporte une chambre 1 sous la forme d'un parallélépipède rectangle ayant une extrémité ouverte et une extrémité fermée.

L'extrémité ouverte est placée sur une embase 2 destinée à faciliter la mise en place de la chambre 1. L'embase 2 est métallique et de dimension adaptée à recevoir la chambre 1. L'embase a un profil en Y dont une branche inférieure est plantée dans le sol S, constituant la surface à étudier, et dont deux branches supérieures reçoivent entre elles l'extrémité ouverte de la chambre 1. Un joint d'isolation 21 est placé entre la chambre 1 et l'embase 2, de manière à rendre sensiblement hermétique l'ensemble de la chambre 1, de l'embase 2 et du sol S.

La chambre 1 recouvre une surface S1 de quelques dixièmes de mètre carré. La surface recouverte est typiquement comprise entre un dixième et dix dixièmes de mètre carré, et vaut 0,25 m² selon un mode de réalisation préféré.

La chambre 1, mise en place sur le sol S, définit un volume au-dessus de la surface à étudier Si. La chambre 1 accumule les gaz émis par la surface S1.

Selon des variantes de réalisation, l'embase et la chambre ne forment qu'une seule pièce, ou la chambre est cylindrique et l'embase circulaire.

Un moyen de circulation des gaz, ici un conduit 3 comporte une première portion 31 qui relie la chambre 1 à une pompe de circulation de gaz 4. Une seconde portion 32 du conduit 3 relie la pompe 4 à un analyseur 5 et une troisième portion 33 du conduit 3 relie l'analyseur 5 à la chambre 1. Le dispositif forme ainsi un circuit fermé de circulation de gaz, externe à la chambre 1.

Le sens de circulation de gaz, imposé par la pompe 4, est schématisé par des flèches. Les gaz sont captés dans la chambre 1, circulent dans la portion 31 du conduit 3 jusqu'à la pompe 4, puis dans la portion 32 jusqu'à l'analyseur 5 et enfin dans la portion 33 pour retourner dans la chambre 1.

La pompe 4 et l'analyseur 5 sont classiques. La pompe 4 peut être intégrée à l'analyseur 5.

L'analyseur 5 doit restituer en sortie la quasi-totalité des gaz qui pénètrent dans l'analyseur, en quantité et en nature.

La première portion 31 du conduit 3 s'étend entre une extrémité 34 située sensiblement au centre de la chambre 1 et la pompe 4. L'extrémité 34 est fermée. Entre l'extrémité 34 et la paroi latérale de la chambre 1, traversée par le conduit 3 via un orifice 36, la première portion 31 est perforée par une pluralité de trous 35 permettant un prélèvement réparti des gaz dans la chambre 1.

Les gaz présents dans la chambre 1 passent dans le conduit 3 par l'intermédiaire des trous 35 de la portion perforée 31, c'est-à-dire qu'ils sont captés dans l'espace intérieur de la chambre 1,

La troisième portion 33 du conduit 3 débouche à l'intérieur de la chambre, par un orifice de sortie 37 situé dans la paroi latérale, sensiblement en face de l'orifice 36. Les gaz qui ont circulé dans le conduit 3 retournent dans la chambre 1 par l'orifice 37.

Un moyen de brassage comporte un ventilateur 6 actionné par un moteur électrique 7. Le ventilateur 6 est placé dans une zone supérieure de l'espace intérieur de la chambre 1, au-dessus de la portion de prélèvement des gaz 31 et de l'orifice 37 du conduit 3. Le ventilateur 6 tourne à faible vitesse de manière à provoquer un brassage des gaz contenus dans la chambre 1, sans toutefois provoquer de turbulence dans la chambre 1.

Un trou 8 de faible diamètre est ménagé dans la paroi supérieure de la chambre 1, de manière à former un évent et à éviter, ou au moins rendre négligeable, une surpression pouvant se produire dans la chambre 1, tout en évitant une fuite excessive de gaz.

Pour mesurer le flux surfacique de gaz émis par le sol, la chambre est disposée sur le sol comme représenté à la figure 1, de manière à former un ensemble sensiblement hermétique. La pompe de circulation 4 est alors actionnée pour faire circuler les gaz au travers de l'analyseur 5 pendant une durée prédéterminée, n'excédant pas quelques minutes. Les gaz émis par le sol s'accumulent dans la chambre et leur concentration est alors analysée de manière classique par l'analyseur 5. L'analyseur 5 détermine la variation des concentrations de gaz en fonction du temps, ce qui permet de calculer le flux surfacique de gaz, connaissant le volume de la chambre 1 et la surface S1. La nature des gaz analysables et la plage de flux mesurable dépendent de l'analyseur 5.

Il est possible de prévoir un moyen d'enregistrement 51 associé à l'analyseur 5, tel qu'un micro-ordinateur, pour enregistrer les valeurs fournies par l'analyseur 5 et pour effectuer des traitements sur ces valeurs.

Les flux mesurés sont généralement faibles, et toute modification de l'environnement, telle que la mise en place du dispositif selon l'invention, altère l'émission de gaz. Des expérimentations ont néanmoins montré que l'influence du dispositif selon l'invention sur le résultat de la mesure est très limitée, et inférieure à celle de dispositifs connus. Ainsi, des facteurs correctifs peuvent être appliqués aux mesures réalisées, pour tenir compte de l'influence du dispositif de mesure sur le flux mesuré.

Les inventeurs ont déterminé expérimentalement que les facteurs correctifs dépendent essentiellement de la nature du sol et de la valeur du flux de gaz. L'influence du dispositif de mesure est d'autant plus importante que le sol est perméable et que la valeur de flux est grande. En pratique, la correction à apporter est une multiplication de la valeur mesurée par un facteur correctif sensiblement compris entre 1 et 1,5.

Les inventeurs ont également déterminé expérimentalement les autres paramètres de fonctionnement du dispositif, tels que le débit de gaz dans le conduit 3, la durée de la mesure, la dimension des différents éléments du dispositif.

Pour mesurer les flux de gaz émis par un terrain de grande dimension, tel qu'une décharge d'ordures ménagères, le dispositif est mis en place successivement en une pluralité d'emplacements qui constituent un échantillonnage du terrain à étudier. Une mesure est réalisée pour chaque emplacement.

Après un traitement mathématique, fondé sur des méthodes géostatistiques classiques, il est possible de fournir notamment la valeur moyenne du flux de gaz pour le terrain considéré ou pour une partie de ce dernier, ou bien la répartition spatiale du flux et de la composition du gaz, ou encore une carte isométrique de flux de gaz.

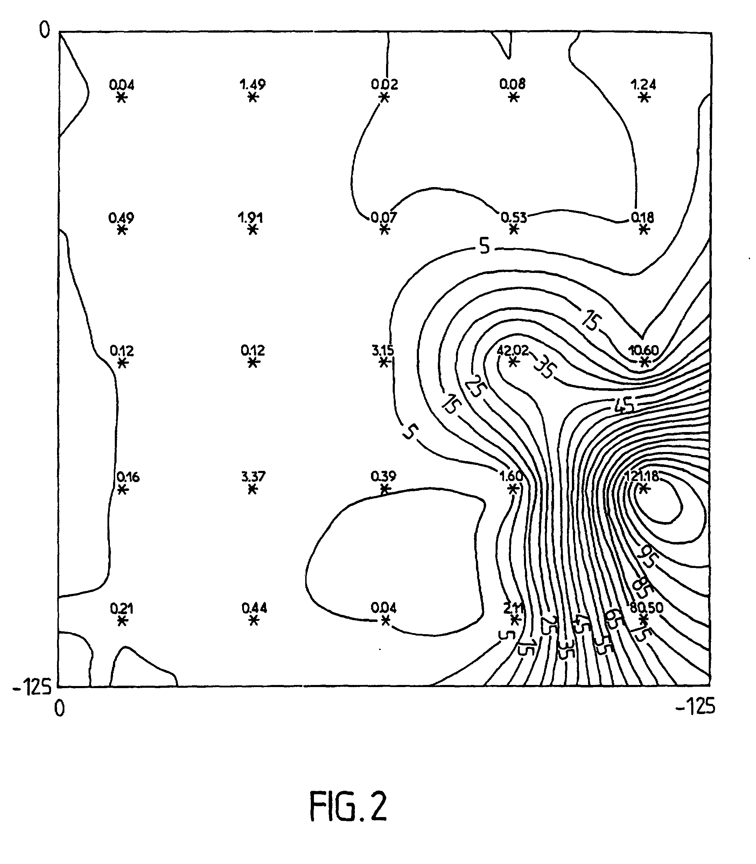

La **figure 2** représente un exemple de relevé de valeurs mesurées grâce au dispositif selon l'invention et une carte de lignes reliant des points présentant le même flux gazeux, réalisée à partir de ces valeurs.

Le terrain étudié est un carré de 125 mètres de côté dans une décharge d'ordures ménagères. Des points de mesure, au nombre de 25, représentés chacun par une étoile, sont régulièrement répartis dans le terrain considéré. La valeur de flux surfacique de méthane mesurée à chaque point et exprimée en cm³.min⁻¹.m⁻², aux conditions normales de température et de pression, est indiquée au-dessus du point. La valeur moyenne de flux mesuré pour le terrain considéré est 11,3 cm³.min⁻¹.m⁻², aux conditions normales de température et de pression.

Les lignes reliant des points ayant le même flux surfacique ont été déterminées par traitement géostatistique des valeurs mesurées. Les lignes représentées vont de 5 en 5, entre 0 est 115 cm³.min⁻¹.m⁻² aux conditions normales de température et de pression.

## Revendications

1. Dispositif de mesure de flux de gaz émis par une surface (S1), comportant une chambre (1) recouvrant ladite surface, délimitant un volume fermé par ladite surface et reliée à un analyseur de gaz (5), **caractérisé en ce qu**'il comporte un moyen (3) de circulation des gaz contenus dans ladite chambre, formant un circuit en boucle fermé (31, 32, 33) entre la chambre et l'analyseur, et en ce que le moyen de circulation comporte un conduit de circulation (3) entre une entrée formée par une portion perforée de conduit (31) s'étendant dans la chambre (1) et une sortie (37) débouchant dans la chambre, via une pompe de circulation de gaz (4) et l'analyseur (5).

2. Dispositif selon la revendication 1, **caractérisé en ce que** la portion perforée (35) s'étend entre le centre de la chambre et une paroi latérale de la chambre (1), et en ce que la sortie (37) est située dans une paroi latérale en vis à vis de l'entrée.

3. Dispositif selon la revendication 1 ou 2, **caractérisé en ce que** la portion perforée (35) s'étend en regard de la surface (S1).

4. Dispositif selon l'une quelconque des revendications 1 à 3, **caractérisé en ce qu**'il comporte un moyen (6, 7) de brassage des gaz contenus dans ladite chambre (1).

5. Dispositif selon la revendication 4, **caractérisé en ce que** le moyen de brassage comporte un ventilateur (6) actionné par un moteur (7).

6. Dispositif selon les revendications 1 et 4 ou 5, **caractérisé en ce que** la portion perforée (35) est située entre le moyen de brassage (6,7) et la surface (S1).

7. Dispositif selon l'une quelconque des revendications 1 à 6, **caractérisé en ce que** la chambre (1) comporte un trou (8) de communication avec l'extérieur.

8. Dispositif selon la revendication 7, **caractérisé en ce que** le trou (8) est situé dans une paroi de la chambre opposée à la surface (S1).

9. Procédé de mesure de flux de gaz émis par une surface (S1), comportant l'étape de disposer une chambre recouvrant ladite surface, délimitant un volume fermé par ladite surface et reliée à un analyseur de gaz (5) **caractérisé en ce qu**'il comporte l'étape de faire circuler les gaz contenus dans ladite chambre, selon un circuit en boucle fermée (31, 32, 33) entre la chambre et l'analyseur, au moyen du dispositif selon l'une quelconque des revendications 1 à 8.

10. Procédé selon la revendication 9, **caractérisé en ce qu**'il comporte l'application d'un facteur correctif aux mesures réalisées.

## Patentansprüche

1. Vorrichtung zur Messung eines von einer Oberfläche (S1) abgegebenen Gasstroms, mit einer die Oberfläche bedeckenden Kammer (1), die ein von der Oberfläche eingeschlossenes Volumen begrenzt und an eine Gasanalyseeinrichtung (5) angeschlossen ist, **dadurch gekennzeichnet**, daß sie eine Einrichtung (3) zur Zirkulation der in der Kammer enthaltenen Gase aufweist, die einen geschlossenen Kreislauf (31, 32, 33) zwischen der Kammer und der Analyseeinrichtung ausbildet, und daß die Zirkulationseinrichtung eine Zirkulationsleitung (3) umfaßt, die zwischen einem Einlaß, der durch einen mit Öffnungen versehenen Leitungsabschnitt (31) gebildet wird und sich in der Kammer (1) erstreckt, über eine Gasumlaufpumpe (4) und die Analyseeinrichtung (5) und einem Auslaß (37) verläuft, der in die Kammer einmündet.

2. Vorrichtung nach Anspruch 1, **dadurch gekennzeichnet**, daß der mit Öffnungen versehene Abschnitt (35) zwischen der Mitte der Kammer und einer Seitenwand der Kammer (1) verläuft, und daß der Auslaß (37) in einer dem Einlaß gegenüberliegenden Seitenwand liegt.

3. Vorrichtung nach Anspruch 1 oder 2, **dadurch gekennzeichnet**, daß der mit Öffnungen versehene Abschnitt (35) der Oberfläche (S1) gegenüberliegend verläuft.

4. Vorrichtung nach einem der Ansprüche 1 bis 3, **dadurch gekennzeichnet**, daß sie eine Umwälzeinrichtung (6, 7) für die in der Kammer (1) enthaltenen Gase umfaßt.

5. Vorrichtung nach Anspruch 4, **dadurch gekennzeichnet**, daß die Umwälzeinrichtung einen über einen Motor (7) betätigten Ventilator (6) aufweist.

6. Vorrichtung nach den Ansprüchen 1 und 4 oder 5, **dadurch gekennzeichnet**, daß der mit Öffnungen versehene Abschnitt (35) zwischen der Umwälzeinrichtung (6, 7) und der Oberfläche (S1) liegt.

7. Vorrichtung nach einem der Ansprüche 1 bis 6, **dadurch gekennzeichnet**, daß die Kammer (1) ein Verbindungsloch (8) zur Außenseite aufweist.

8. Vorrichtung nach Anspruch 7, **dadurch gekennzeichnet**, daß das Loch (8) in einer der Oberfläche (S1) gegenüberliegenden Wand der Kammer angeordnet ist.

9. Verfahren zur Messung eines von einer Oberfläche (S1) abgegegebenen Gasstroms, mit dem Schritt des Anbringens einer die Oberfläche bedeckenden Kammer, die ein von der Oberfläche eingeschlossenes Volumen begrenzt und an eine Gasanalyseeinrichtung (5) angeschlossen ist, **dadurch gekennzeichnet**, daß es den Schritt umfaßt, bei dem man die in der Kammer enthaltenen Gase in einem geschlossenen Kreislauf (31, 32, 33) zwischen der Kammer und der Analyseeinrichtung mittels der Vorrichtung nach einem der Ansprüche 1 bis 8 zirkulieren läßt.

10. Verfahren nach Anspruch 9, **dadurch gekennzeichnet**, daß es die Anwendung eines Korrekturfaktors auf die durchgeführten Messungen umfaßt.

## Claims

1. Device for measuring gas flows emitted by a surface (S1), having a chamber (1) covering the said surface, delimiting a volume closed by the said surface and connected to a gas analyser (5), **characterised in that** it has a means (3) for circulating the gases contained in the said chamber, forming a circuit in a closed loop (31, 32, 33) between the chamber and the analyser, and in that the circulation means includes a circulation conduit (3) between an inlet formed by a perforated conduit portion (31) extending into the chamber (1) and an outlet (37) opening out into the chamber, via a gas circulation pump (4) and the analyser (5).

2. Device according to Claim 1, **characterised in that** the perforated portion (35) extends between the centre of the chamber and a side wall of the chamber (1), and in that the outlet (37) is situated in a side wall opposite the inlet.

3. Device according to Claim 1 or 2, **characterised in that** the perforated portion (35) extends opposite the surface (S1).

4. Device according to any one of Claims 1 to 3, **characterised in that** it has a means (6, 7) for stirring the gases contained in the said chamber (1).

5. Device according to Claim 4, **characterised in that** the stirring means includes a fan (6) actuated by a motor (7).

6. Device according to Claims 1 and 4 or 5, **characterised in that** the perforated portion (35) is situated between the stirring means (6, 7) and the surface (S1).

7. Device according to any one of Claims 1 to 6, **characterised in that** the chamber (1) has a hole (8) for communicating with the outside.

8. Device according to Claim 7, **characterised in that** the hole (8) is situated in a wall of the chamber opposite to the surface (S1).

9. Method of measuring gas flows emitted by a surface (S1), including the step of disposing a chamber covering the said surface, delimiting a volume closed by the said surface and connected to a gas analyser (5), **characterised in that** it includes the step of causing the gases contained in the said chamber to circulate, in a circuit in a closed loop (31, 32, 33) between the chamber and the analyser, by means of the device according to any one of Claims 1 to 8.

10. Method according to Claim 9, **characterised in that** it includes the application of a correction factor to the measurements made.
